(19) Europäisches Patentamt
European Patent Office
Office européen des brevets

(11) Publication number: **0 081 859 A2**

# (12) EUROPEAN PATENT APPLICATION

(21) Application number: **82111791.8**

(22) Date of filing: **04.07.80**

(51) Int. Cl.[3]: **C 07 D 233/60**

(30) Priority: **06.07.79 JP 86249/79**

(43) Date of publication of application: **22.06.83 Bulletin 83/25**

(84) Designated Contracting States: **DE FR GB**

(60) Publication number of the earlier application in accordance with Art. 76 EPC: **0031388**

(71) Applicant: **SMITHKLINE BECKMAN CORPORATION, 1 Franklin Plaza, Philadelphia Pennsylvania 19101 (US)**

(72) Inventor: **Teraji, Tsumotu, 20-6, Kofudai 6-chome Toyono-cho, Toyono-gun Osaka 563-01 (JP)**
Inventor: **Nakai, Yoshiharu, 7-18, Zeze 1-chome, Otsu-shi Shiga 520 (JP)**
Inventor: **Durant, Graham John, 401 Knightsfield, Welwyn Garden City Herfordshire (GB)**

(74) Representative: **Hargreaves, Gerald Henry, Dr. et al, SMITH KLINE & FRENCH Laboratories Limited Patent Department Mundells, Welwyn Garden City Hertfordshire AL7 1EY (GB)**

## (54) Process for preparing imidazole derivatives.

(57) Imidazole derivatives including cimetidine having histamine-$H_2$ antagonist activity and of the formula

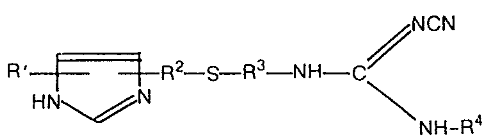

where $R^1$ and $R^4$ are each lower alkyl, and $R^2$ and $R^3$ are each lower alkylene, are prepared by reacting a compound of the formula

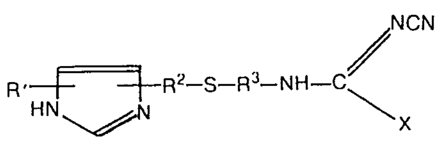

where X is halogen, mercapto, cyanamino, an aromatic 5-membered N-containing heterocycle-N-yl group or a group $-S(O)_n-Z$ in which Z is hydroxy or alkyl and n is 1 or 2.

EP 0 081 859 A2

0081859

SPECIFICATION

PROCESS FOR PREPARING IMIDAZOLE DERIVATIVES

This invention relates to a process for preparing imidazole derivatives of the formula

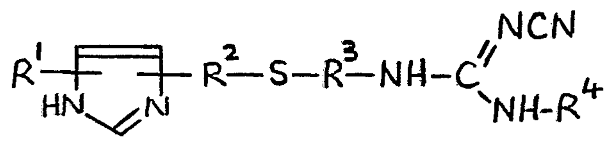 (I)

where $R^1$ and $R^4$ are each lower alkyl, and $R^2$ and $R^3$ are each lower alkylene, and their salts.

Imidazole derivatives of the formula (I) and their salts, which include cimetidine, are compounds having histamine $H_2$-antagonist activity and useful as anti-ulcer agents.

According to the invention a process for preparing an imidazole derivative of formula (I) comprises reacting a corresponding compound of the formula (II)

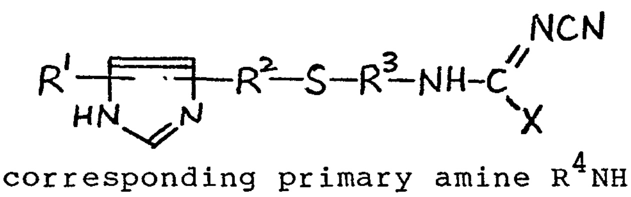

with a corresponding primary amine $R^4NH_2$, characterised in that X is halogen, mercapto, cyanamino, an aromatic 5-membered N-containing heterocycle-N-yl group or a group $-S(O)_n-Z$ in which Z is hydroxy or alkyl, and n is 1 or 2.

In the starting materials each of $R^1$ and $R^4$ may be a straight or branched alkyl group having 1 to 6 carbon atoms such as methyl, ethyl, propyl, isopropyl, butyl, isobutyl, tert-butyl, pentyl or hexyl, and each of $R^2$ and $R^3$ may be a straight or branched alkylene group having 1 to 6 carbon atoms such as methylene, ethylene, trimethylene, tetramethylene, pentamethylene, hexamethylene, 2-methyl-trimethylene or 2,2-dimethyl-

0081859

trimethylene. $R^2$ and $R^3$ may be the same or different from each other. When X is halogen, it may be chlorine, bromine or iodine. When X is an aromatic 5-membered N-containing heterocycle-N-yl group, it may be 1-pyrazolyl, 1-imidazolyl, 1-pyrrolyl, 1,2,3-triazol-1-yl, 3,5-dimethyl-1-pyrazolyl, or 1,2,4-triazol-4-yl. When X is a group $-S(O)_n-Z$, it may be sulfino, sulfo, alkylsulfinyl having 1 to 6 carbon atoms (e.g. methylsulfinyl, ethylsulfinyl, propylsulfinyl, isopropylsulfinyl, butylsulfinyl, isobutylsulfinyl, tert-butylsulfinyl, pentylsulfinyl and hexylsulfinyl), or alkylsulfonyl having 1 to 6 carbon atoms (e.g. methylsulfonyl, ethylsulfonyl, propylsulfonyl, isopropylsulfonyl, butylsulfonyl, isobutylsulfonyl, tert-butylsulfonyl, pentylsulfonyl and hexylsulfonyl).

The reaction can be carried out in a conventional solvent such as alcohol (e.g. methanol, ethanol, propanol, butanol and isopropyl alcohol), acetonitrile, methylene dichloride, dimethylformamide, dimethylsulfoxide, dioxane and tetrahydrofuran. The reaction can be carried out at room temperature or with cooling.

Where a compound (II) in which X is halogen or a sulfino or sulfo group is used as starting material, the reaction is preferably carried out in the presence of a base such as triethylamine, pyridine, N-methylaniline and 1,5-diazabicyclo[5.4.0]undec-5-ene.

Where a compound (II) in which X is a mercapto group is used as starting material, the reaction is preferably carried out in the presence of a compound of a heavy metal (e.g. mercury, silver and lead).

Where a compound (II) in which X is a sulfino or sulfo group is used, it can be in the form of a salt such as a salt of an alkali metal (e.g. sodium and potassium) or a 1,5-diazabicyclo[5.4.0]undec-5-ene (DBU) salt.

The imidazole derivative (I) produced by a process of this invention may be isolated and purified, and if

desired, converted into its salt such as a hydrochloride in a conventional manner.

The starting materials used in the process of this invention can be prepared by methods described in the following Examples or by similar methods, or by methods similar to those described in European Patent Specification 80901220.6.

The following Examples illustrate the preparation of cimetidine by the process of the invention.

EXAMPLE 1

(i) To a solution of sodium metal (1.01 g) in ethanol (120 ml) were added cyanamide (18.5 g) and N-cyano-S-methyl-N'-[2-(5-methylimidazol-4-ylmethyl-thio)ethyl]-isothiourea (10.76 g). The resulting mixture was heated under reflux for 1.5 hr. After concentration under reduced pressure, the residue was added to cold water (50 ml), acidified (pH 4.5) with 10% hydrochloric acid and cooled for 1 hr in an ice bath. The precipitate was filtered, washed with water and dried. The resulting crude product (7.43 g) was recrystallised from 50% aqueous ethanol to give N,N'-dicyano-N"-[2-(5-methyl-imidazol-4-ylmethylthio)ethyl]guanidine (5.17 g), yield 49.2%, m.p. 183-186°C. (Calc. for $C_{10}H_{13}N_7S$: C, 45.61; H, 4.98; N, 37.24; S, 12.18. Found: C, 45.59; H, 4.99; N, 36.51; S, 12.17.)

IR (nujol) $\nu$ max: 3330, 3110, 2660, 2170, 1575, 1518, 1435, 1378, 1348, 1310, 1230, 867 $cm^{-1}$.

NMR ($DMSO-d_6+D_2O$): $\delta$ 2.32 (s, 3H), 2.4 - 2.7 (m, 2H), 3.26 (t, J=7Hz, 2H), 3.83 (s, 2H), 8.77 (s, 1H).

(ii) N,N'-Dicyano-N"-[2-(5-methylimidazol-4-ylmethylthio)-ethyl]guanidine (527 mg) was added to a solution of methylamine in ethanol (40%, 3.1 g) and the mixture stirred for 47 hr at room temperature. After concentration under reduced pressure, the residue was

triturated with acetonitrile. The insoluble starting material was filtered off and the filtrate was concentrated under reduced pressure and chromatographed on silica [eluant: $CH_3CN$-$CH_3OH$ (4:1)]. The resulting crude product was purified by preparative thin layer chromatography ($SiO_2$, $CH_3COOC_2H_5$-$CH_3OH$- 28% aq. $NH_3$ (10:1:1)] and recrystallised from acetonitrile to give N-cyano-N'methyl-N"-[2-(5-methylimidazol-4-ylmethylthio)-ethyl]-guanidine (1.0 mg), m.p. 135-137°C.

EXAMPLE 2

(i) A solution of 2-(5-methylimidazol-4-yl-methylthio)-ethylisothiocyanate [obtained from its hydrochloride (4.17 g)] in chloroform (20 ml) was added to a solution of cyanamide (1.05 g) and DBU (3.80 g) in chloroform (50 ml) and stirred overnight. After concentration, the residue was dissolved in water (30 ml) and washed with ether, decolorized with charcoal and acidified to pH 5 with 10% hydrochloric acid under ice-cooling. The precipitate was filtered, washed with water and dried to give N-cyano-N'-[2-(5-methylimidazol-4-ylmethylthio)-ethyl]-thiourea monohydrate (3.57 g), yield 78.1%, m.p. 158-161°C. (Calc. for $C_9H_{13}N_5S_2.H_2O$: C, 39.54; H, 5.53; N, 25.62; S, 23.46. Found: C, 39.71; H, 5.44; N, 25.69; S, 22.73.

IR (nujol) $\nu$ max: 3400, 3270, 3130, 2760, 2650, 2190, 1652, 1540, 1470, 1430, 1381, 1362, 1278, 1237, 1217, 1205, 1103, 1088, 967, 837 $cm^{-1}$.

NMR (DMSO-$d_6$+$D_2O$): $\delta$ 2.32 (s, 3H), 2.4 - 2.8 (m, 2H), 3.0 - 3.67 (m, 2H), 3.87 (s, 2H), 8.93 (s, 1H).

(ii) To a suspension of N-cyano-N'-[2-(5-methylimidazol-4-ylmethylthio)ethyl]thiourea monohydrate (765 mg) in methylene dichloride (7 ml) was introduced dry hydrogen chloride for a few minutes under ice-cooling. Phosphorus pentachloride (1.25 g) was added and the mixture was stirred at room temperature for 3 hr. The reaction

mixture containing N'-cyano-N-[2'(5-methylimidazol-4-ylmethylthio)ethyl]-chloroformamidine was concentrated to small volume, diluted with methylene dichloride (5 ml) and cooled to -5°C. To the mixture was added a 40% solution of methylamine in ethanol (15 ml) at below 30°C. After stirring for 20 hr at room temperature, the reaction mixture was filtered and the filtrate was concentrated to small volume, diluted with acetonitrile and filtered again. The filtrate was dried up and the residue was purified by column chromatography [$SiO_2$, $CH_3CN$-$CH_3OH$ (4:1)], preparative thin layer chromatography ($SiO_2$, $CH_3COOC_2H_5$-$CH_3OH$- 28% aq. $NH_3$ (10:1:1)] and then recrystallisation from acetonitrile to give N-cyano-N'-methyl-N"-[2-(5-methyl-imidazol-4-yl-methylthio)ethyl]guanidine (10.4 mg). Yield 1.5%, m.p. 136.5-138°C.

CLAIMS

1. A process for preparing an imidazole derivative of the formula

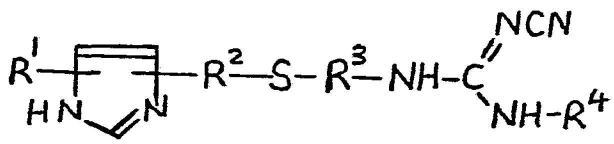

where $R^1$ and $R^4$ are each lower alkyl, and $R^2$ and $R^3$ are each lower alkylene, which comprises reacting a corresponding compound of the formula

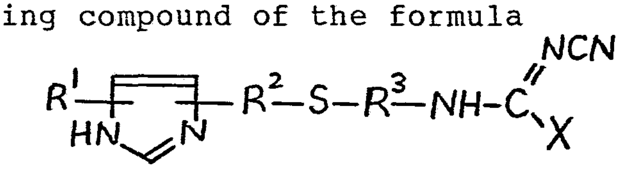

with a primary amine $R^4NH_2$, characterised in that X is halogen, mercapto, cyanamino, an aromatic 5-membered N-containing heterocycle-N-yl group or a group $-S(O)_n-Z$ in which Z is hydroxy or alkyl, and n is 1 or 2.

2. A process according to Claim 1, in which the imidazole derivative is cimetidine.